# EUROPÄISCHE PATENTSCHRIFT

(11) **EP 1 099 454 B1**
(45) Veröffentlichungstag und Bekanntmachung des Hinweises auf die Patenterteilung: **27.08.2003**
(21) Anmeldenummer: 99122533.5
(22) Anmeldetag: 12.11.1999
(51) Int. Cl.: A61M 39/10, F16L 37/248

(54) **Schnellkupplung für ein Medikamenteninfusionssystem**
Connector for an infusion system
Raccord pour système de perfusion

(43) Veröffentlichungstag der Anmeldung: 16.05.2001
(73) Patentinhaber: Clinico GmbH, 36251 Bad Hersfeld (DE)
(72) Erfinder: Heinzerling, Jörg, 36251 Bad Hersfeld (DE)
(74) Vertreter: von Raffay, Vincenz, Dipl.-Ing.

(56) Entgegenhaltungen:
- EP-A- 0 634 190
- EP-A- 0 738 520
- WO-A-91/16938
- WO-A-96/14096
- US-A- 5 178 612

## Beschreibung

Die Erfindung betrifft eine Schnellkupplung nach dem Oberbegriff des Anspruches 1.

Eine derartige Schnellkupplung ist aus der WO 91/16938 bekannt. Bei dieser bekannten Schnellkupplung übernimmt der Bajonettverschluß sowohl die Sicherung gegen axiales Herausziehen als auch die Drehsicherung, d.h. der Bajonettverschluß kann durch Drehen nur wieder gelöst werden, wenn ein bestimmter Widerstand überwunden wird. Dieser Widerstand wird dadurch erreicht, daß die Bajonettvertiefung in der Buchse eine Engstelle aufweist, durch die der Bajonettvorsprung an dem Stecker nur mit entsprechendem Kraftaufwand hindurch bewegbar ist.

Aus der EP 739 222 B1 ist eine entsprechende Schnellkupplung nach dem Oberbegriff des Anspruches 1 bekannt, bei der der Bajonettvorsprung bei der Verdrehung, bevor er in die Bajonettvertiefung gelangt, mit seiner radial vorstehenden Fläche über eine entsprechende Steuerfläche auf der Innenseite der Buchse bewegt wird, wobei die Abmessungen hier so gewählt sind, daß die Reibung zwischen der Stirnfläche des Bajonettvorsprunges und der Steuerfläche relativ groß ist, um den gewünschten Widerstand aufzubringen. Bei den beiden bekannten Schnellkupplungen erfolgt die Sicherung gegen Herausziehen in axialer Richtung und gegen ein unbeabsichtigtes Lösen durch Drehung jeweils durch den Bajonettvorsprung, der also zwei unterschiedliche Aufgaben übernehmen muß.

Der Erfindung liegt nun die Aufgabe zugrunde, eine Schnellkupplung der eingangs genannten Art zu schaffen, bei der die Sicherung gegen axiales Herausziehen und die Drehsicherung unabhängig voneinander erfolgen, so daß die beiden Sicherungsarten entsprechend den an sie gestellten Anforderungen individuell ausgeführt und gestaltet werden können.

Diese Aufgabe wird grundsätzlich durch das Kennzeichen des Anspruches 1 gelöst.

Erfindungsgemäß ist also ein herkömmlicher Bajonettverschluß vorhanden, der durch einen Bajonettvorsprung an dem Stecker und einer entsprechenden Bajonettvertiefung in der Buchse gebildet wird, und eine Sicherung gegen axiales Herausziehen bildet. Die Verdrehsicherung wird durch die gesonderte Rastnase erreicht, die in eine entsprechende Vertiefung in der Buchse einschnappt. Diese Rastnase kann so ausgebildet gestaltet und aus entsprechenden Materialien hergestellt sein, daß sie ihre Aufgabe der Drehsicherung besonders gut erfüllt und gleichzeitig für ein merkliches, ggf. hörbares einschnappen bei Erreichen der Verriegelungsstellung sorgt. Wenn der Patient die beiden Teile, d.h. Buchse und Stecker, der Schnellkupplung miteinander verbinden will, steckt er die beiden Teile ineinander, verdreht diese gegeneinander, so daß der Bajonettvorsprung in die Bajonettvertiefung bewegt wird, um die Sicherung gegen ein Herausziehen in axialer Richtung herzustellen. Das Ende der Drehbewegung, d.h. das Erreichen der sicheren Stellung gegen axiales Herausziehen, wird durch das Einschnappen der Rastnase in die Vertiefung angezeigt. Gleichzeitig ist ein Lösen nur mit entsprechendem Kraftaufwand möglich.

Wenn die Schnellkupplung wie in Anspruch 2 angegeben ausgestaltet ist, dann ist die Buchse mit besonderem "Fleisch" versehen, auf dem die Rastnase in die Verriegelungsstellung gleitet und nach Erreichen der Verriegelungsstellung und nach dem Einschnappen sicher und festgehalten wird.

In besonders vorteilhafter Weise kann die Erfindung so ausgestaltet sein wie in dem Anspruch 3 angegeben. Dadurch, daß in dem Stecker ein besonderer Einsatz aus härterem Kunststoff vorgesehen ist, an dem auch die federnde Rastnase ausgebildet ist, kann diese ebenso wie der Bajonettvorsprung aus entsprechend hartem Material ausgebildet sein, um die gewünschten Sicherungsaufgaben zu erfüllen. Das äußere Gehäuse ist aus weichem Kunststoff, um für einen guten und gleichmäßigen Übergang in den angeschlossenen Schlauch zu sorgen. Entsprechendes gilt für die Buchse, wobei das weiche Material der Buchse gewisse "Schmiereigenschaften" aufweist, die für eine leichte und glatte Bewegung des Bajonettvorsprunges in der Bajonettvertiefung und der Rastnase auf der Gleit- und Anschlagfläche in die Vertiefung in der Buchse sorgen. Die Rastnase und die wesentlichen sie tragenden Teile bestehen aus einem anderen Material als die Buchse und das Gehäuse des Steckers. An die Buchse und an das Gehäuse werden die Schläuche zur Verbindung mit der Pumpe und dem Patienten angeschlossen. Das weiche Material sorgt für einen allmählichen Übergang in diesem Anschlußbereich und wirkt einem Abknicken entgegen. Die Verriegelungsteile, nämlich Rastnase und Bajonettvorsprung sind aus verschleißfestem Material. Die federnde Eigenschaft der Rastnase wird durch die Materialeigenschaften unterstützt.

In besonders vorteilhafter Weise ist die Schnellkupplung so ausgebildet wie in den Ansprüchen 5 und 6 definiert. Da die Rastnasen bei dieser symmetrischen Ausbildung auf einem größeren Durchmesser liegen, haben sie zur Übertragung des Drehmomentes einen relativ großen Drehmomentenarm. Weiterhin ist die Ausbildung der flachen Form (Anspruch 6) möglich, die eine gute Handhabung durch den Patienten ermöglicht. Der Patient kann den flachen Stecker und die flache Buchse gut ergreifen, diese in der Offenstellung, d.h. 90° gegeneinander verdreht, zusammenstecken und dann zur Herstellung der Verriegelung gegeneinander verdrehen, bis die beiden flachen Bauteile miteinander fluchten.

Weitere vorteilhafte Ausgestaltungen sind Gegenstand der Ansprüche 7 und 8.

Im folgenden wird die Erfindung unter Hinweis auf die Zeichnung anhand eines Ausführungsbeispieles näher erläutert.

Es zeigt:
- Fig. 1: eine teilweise geschnittene Draufsicht auf eine Ausführungsform einer Schnellkupplung nach der Erfindung;
- Fig. 2: eine stirnseitige Ansicht der Darstellung nach Fig. 3;
- Fig. 3: einen Schnitt durch die Buchse;
- Fig. 4: einen Schnitt durch den aus Gehäuse und Einsatz bestehenden Stecker;
- Fig. 5: eine stirnseitige Ansicht der Darstellung der Fig. 4;
- Fig. 6: einen Schnitt durch Buchse und Stecker in der Stellung, in der sie zusammengesteckt werden;
- Fig. 7: einen Schnitt durch Buchse und Stecker gemäß der Linie B - B der Fig. 1 in der Verriegelungsstellung;
und
- Fig. 8: einen Schnitt durch den Stecker gemäß der Linie A - A der Fig. 5.

Die in der Zeichnung dargestellte Schnellkupplung besteht aus einem Stecker 1 und einer Buchse 3, an deren Enden Schlauchverbindungen vorgesehen sind. Bei dieser Ausführungsform ist der Stecker mit der Patientenseite und die Buchse mit der Pumpenseite verbunden.

Der Stecker 1 ist zweiteilig ausgebildet, d.h. er besteht aus einem Gehäuse 2 aus relativ weichem Kunststoff und einem Einsatz 4 aus relativ hartem Kunststoff. Die Buchse 3 besteht aus dem weichen Kunststoff, aus dem auch das Gehäuse 2 hergestellt ist.

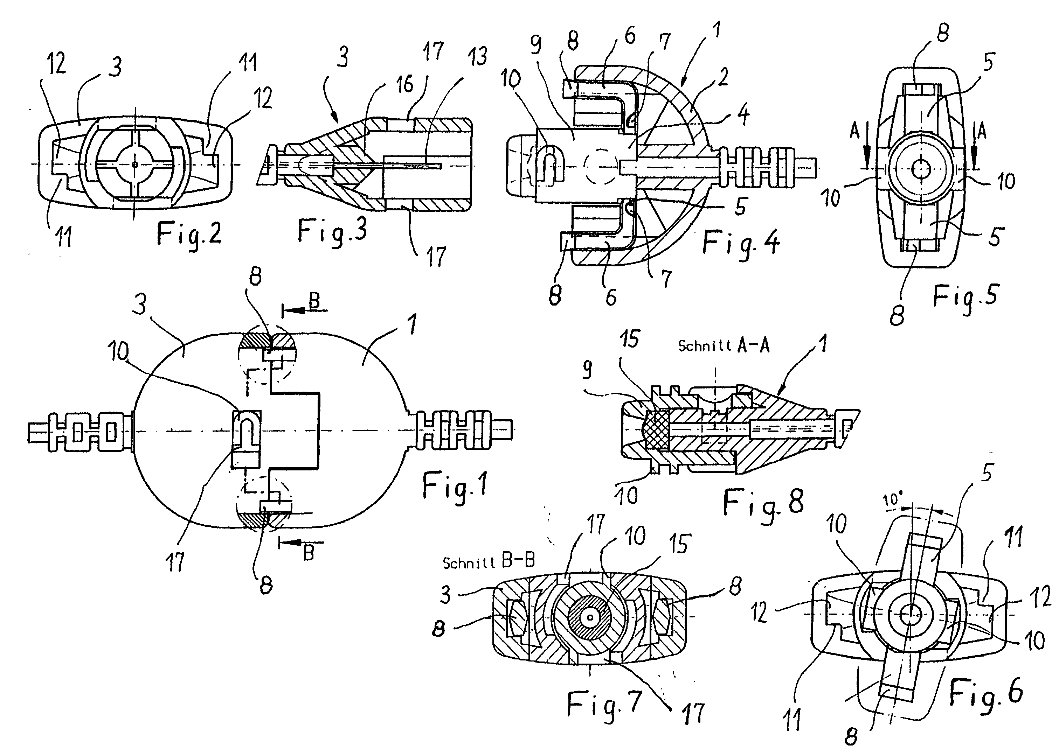

Der Einsatz 4 besteht aus einem zylindrischen Ansatz 9, der auf einem U-förmigen Bügel 5,6 ausgebildet ist. Auf dem Ansatz befinden sich einander gegenüberliegend zwei Bajonettvorsprünge 10. In dem Ansatz ist eine Durchgangsöffnung für das zu verabreichende Medikament vorgesehen, in der sich eine Dichtscheibe 15 befindet. In diese Durchgangsöffnung wird beim Herstellen der Verbindung die in Fig. 3 gezeigte Nadel 13 eingeführt, die mit der Pumpe verbunden ist. Ein dachförmiger Vorsprung 16 der die Nadel 13 trägt, gelangt hierbei an der Stirnfläche des Ansatzes 9 im Bereich der Öffnung zur Anlage.

Der Bügel, auf dem der Ansatz 9 angeordnet ist und der in das Gehäuse 2 eingesetzt ist, weist zwei Schenkel 6 mit Rastnasen 8 auf, die dachförmig gestaltet sind, wobei der First der Buchse zugekehrt ist. In dem Steg 5 des Bügels sind Kerben 7 vorgesehen, die eine federnde Bewegung der Stege 6 mit den Rastnasen 8 in Achsrichtung ermöglichen.

Die Buchse 3 weist eine Öffnung oder Vertiefung auf, die einen rechteckigen Querschnitt hat und in die der Ansatz 9 paßt und eingeführt werden kann. In der Außenwand der Buchse befinden sich zwei Bajonettvertiefungen 17, die als Fenster ausgebildet sind. Wie in Fig. 2 erkennbar, ist im Bereich der Stirnfläche der Buchse, die dem Stecker zugekehrt ist, auf jeder Seite eine Gleit- oder Anschlagfläche 11 ausgebildet, die, wie später noch beschrieben, der Führung und der Sicherung der zugeordneten Rastnase dient.

Wenn die Schnellkupplung aus Stecker und Buchse durch einen Patienten zusammengefügt werden sollen, nehmen die Teile ungefähr die in Fig. 6 gezeigte Stellung ein, d.h. sie sind nicht genau 90° zueinander angeordnet, sondern um ca. 10° bereits in die Richtung gegeneinander verdreht, in die die Verriegelung stattfindet. Hierdurch wird dem Patienten die Drehrichtung vorgegeben.

Stecker und Buchse werden zusammengesteckt, wobei die Nadel 13 durch die Dichtscheibe 15 hindurchgesteckt wird und in die entsprechende Öffnung oder den Kanal in den Stecker eindringt. Der spitze Vorsprung 16 gelangt hierbei auf der Stirnfläche des Ansatzes 9, d.h. im Bereich der Öffnung in diesem Ansatz, zur Anlage. Wenn die beiden Teile vollständig zusammengesteckt sind, erfolgt die Verdrehung um ca. 90°, d.h. eigentlich nur noch 80°. Hierbei werden die Bajonettvorsprünge 10 in die als Fenster ausgebildeten Bajonettvertiefungen 17 bewegt. Und zwar solange, bis die in Drehrichtung vordere Fläche der Bajonettvorsprünge an der entsprechenden Kante des Fensters anschlägt. Im übrigen wird die Drehbewegung aufgrund des rechteckigen Querschnittes der Buchsenöffnung nicht behindert. Gegen Ende der Drehbewegung laufen die Rastnasen 8 auf die Gleit- oder Anschlagfläche 11 an der Stirnfläche der Buchse 3 auf. Aufgrund ihrer dachförmigen Ausbildung werden sie allmählich zurück gedrückt, da sie bedingt durch die Kerben 7 und die relativ große Materialhärte federn. Bei fortgesetzter Drehung in die End- oder Verriegelungsstellung gelangen die Rastnasen 8 hinter die Gleit- und Anschlagflächen 11, wobei bedingt durch die Vorspannung ein Einschnappen erfolgt.

Die Rastnasen 8 liegen auf einem größeren Durchmesser als die Bajonettvorsprünge 10. Der Hebelarm, der den Drehmoment festlegt, ist relativ groß, so daß eine exakte und einfühlsame Steuerung der Ver- und Entriegelung möglich ist. Auch ist eine relativ flache Ausbildung der Schnellkupplung, d.h. der Buchse 3 und des Gehäuses 2 mit dem Einsatz 4 möglich. Hierdurch ist es für den Patienten leicht und auch unter Sicherheitsgesichtspunkten einfach, die Entriegelungsstellung zu erkennen, die beiden Teile zusammenzustecken und dann gegeneinander zu verdrehen, bis die in Fig. 1 gezeigte insgesamt flache und glatte fluchtende Verriegelungsstellung der beiden Teile eingenommen wird. Es ist nicht nur eine gute Handhabe möglich, sondern es ist auch gut erkennbar, ob die Verriegelungsstellung eingenommen wird. Gleichzeitig wird das Einnehmen der Verriegelungsstellung durch das Einschnappen der Rastnasen 8 in die Vertiefungen 12 deutlich fühlbar und ggf. hörbar.

Wenn die Schnellkupplung wieder gelöst werden soll, erfolgt die Drehbewegung in die entgegengesetzte Richtung. Durch die dachförmige Ausbildung der Rastnasen werden diese gegen die Federkraft zurück gedrückt und gleiten auf der zugehörigen Gleit- und Anschlagfläche 11. Gleichzeitig wird der Bajonettverschluß gelöst. Wenn die in Fig. 6 gezeigte Stellung erreicht ist, können die Teile auseinandergezogen werden.

Die zweiteilige Ausbildung des Steckers ermöglicht die Materialauswahl dieser beiden Teile entsprechend der ihnen zugedachten technischen Aufgaben.

## Patentansprüche

1. Schnellkupplung für ein Medikamenteninfusionssystem mit einer stirnseitig offenen Buchse (3) und einem Stecker (1), die durch einen Bajonettverschluß durch Verdrehen gegeneinander um ca. 90° miteinander verbindbar sind, **dadurch gekennzeichnet, daß** der Stecker (1) eine Rastnase (8) aufweist, die in Achsrichtung senkrecht zur Drehebene federt und in der Verriegelungsstellung in eine zugeordnete Vertiefung (12) der Buchse (3) einschnappt.

2. Schnellkupplung nach Anspruch 1, **dadurch gekennzeichnet, daß** die Vertiefung (12) eine zusätzliche Gleit- oder Anschlagfläche (11) für die Rastnase (8) aufweist.

3. Schnellkupplung nach Anspruch 1 oder 2, **dadurch gekennzeichnet, daß** der Stecker (1) ein äußeres Gehäuse aus relativ weichem Kunststoff, aus dem auch die Buchse (3) besteht aufweist, so daß einem Abknicken im Übergangsbereich zu den ungeschlossenen Schläuchen entgegengewirkt wird, und einen Einsatz (4) aus relativ hartem Kunststoff aufweist, der die federnde Eigenschaft der Rastnase unterstützt, an welchem Einsatz die federnde Rastnase (8) und der Bajonettvorsprung (10), der in der Verriegelungsstellung in einem Bajonettfenster (17) der Buchse (3) liegt, ausgebildet sind.

4. Schnellkupplung nach Anspruch 1, 2 oder 3, **dadurch gekennzeichnet, daß** die Rastnase (8) an einem federnden Bügel (5) vorgesehen ist.

5. Schnellkupplung nach Anspruch 3 oder 4, **dadurch gekennzeichnet, daß** der Einsatz (4) sowohl zwei symmetrisch einander gegenüberliegend angeordnete Bajonettvorsprünge (10) als auch zwei Rastnasen (8) aufweist,
wobei die Rastnasen und die Bajonettvorsprünge um ca. 90° gegeneinander versetzt sind und
die Rastnasen auf einem größeren Durchmesser als die Bajonettvorsprünge liegen.

6. Schnellkupplung nach Anspruch 5, **dadurch gekennzeichnet, daß** das äußere Gehäuse (2) des Steckers (1) und die Buchse (3) derart flach ausgebildet sind, daß sie in der Verriegelungsstellung eine flache, glatt ineinander übergehende Form aufweisen und
daß die Rastnasen (8) und die zugeordneten Vertiefungen (12) auf dem größeren Durchmesser der flachen Form liegen.

7. Schnellkupplung nach Anspruch 5 oder 6, **dadurch gekennzeichnet, daß** die symmetrisch einander gegenüberliegenden Rastnasen (8) an den Enden der Schenkel (6) eines U-förmigen Bügels (5,6) des Einsatzes (4) vorgesehen sind und
daß der Steg (5) des U-förmigen Bügels Kerben (7) zur Federung der Rastnasen aufweist.

8. Schnellkupplung nach einem der vorstehenden Ansprüche, **dadurch gekennzeichnet, daß** die Rastnase (8) eine dachförmige Gestalt aufweist, deren First der Vertiefung der Buchse (3) zugekehrt ist.

## Claims

1. Quick coupling for a medicament infusion system with a frontally open socket (3) and a plug (1), which are interconnectable by a bayonet catch by turning against one another by approximately 90°, **characterized in that** the plug (1) has a detent (8), which is elastic in the axial direction perpendicular to the rotation plane and in the locking position snaps into an associated depression (12) of the socket (3).

2. Quick coupling according to claim 1, **characterized in that** the depression (12) has an additional sliding or stop face (11) for the detent (8).

3. Quick coupling according to claim 1 or 2, **characterized in that** the plug (10 has an external casing made from a relatively flexible plastic from which the socket (3) is also made, so that a bending in the transition region to the connected tubes is counteracted and also has an insert (4) made from a relatively rigid plastic assisting the elastic character of the detent and on said insert are formed the elastic detent (8) and bayonet projection (10), which in the locking position is located in a bayonet window (17) of the socket (3).

4. Quick coupling according to claims 1, 2 or 3, **characterized in that** the detent (8) is provided on an elastic clip (5).

5. Quick coupling according to claim 3 or 4, **characterized in that** the insert (4) has two symmetrically facing bayonet projections (10) and also two detents (8), the detents and the bayonet projections being mutually displaced by approximately 90° and the detents are located on a larger diameter than the bayonet projections.

6. Quick coupling according to claim 5, **characterized in that** the outer casing (2) of the plug (1) and the socket (3) are flat in such a way that in the locking position they have a flat shape passing into one another and that the detents (8) and associated depressions (12) are located on the larger diameter of the flat shape.

7. Quick coupling according to claim 5 or 6, **characterized in that** the symmetrically facing detents (8) are provided on the ends of the legs (6) of a U-shaped clip (5, 6) of insert (4) and the crosspiece (5) of the U-shaped clip has notches for the elastic action of the detents.

8. Quick coupling according to one of the preceding claims, **characterized in that** the detent (8) has a roof-shaped design, whose ridge faces the depression of the socket (3).

## Revendications

1. Raccord rapide pour un système de perfusion destiné à l'administration de médicaments, comprenant un embout femelle (3) ouvert du côté frontal et un embout mâle (1), qui peuvent être assemblés l'un à l'autre par un emboîtement à baïonnette par une rotation sur 90° l'un par rapport à l'autre, **caractérisé en ce que** l'embout mâle (1) comporte un téton de blocage (8), qui fait ressort dans le sens axial perpendiculairement au plan de rotation et qui, dans la position de verrouillage, s'enclenche dans une cavité (12) correspondante dans l'embout femelle (3).

2. Raccord rapide selon la revendication 1, **caractérisé en ce que** la cavité (12) comporte une face de glissement ou de butée (11) supplémentaire pour le téton de blocage (8).

3. Raccord rapide selon la revendication 1 ou 2, **caractérisé en ce que** l'embout mâle (1) comporte un boîtier extérieur réalisé en matière plastique relativement souple, dans laquelle est également formé l'embout femelle (3), de manière à empêcher une rupture par pliage dans la zone de transition vers les tubulures raccordées, et un insert (4) qui est réalisé dans une matière plastique relativement dure favorisant la propriété de ressort du téton de blocage et contre lequel sont formés les tétons de blocage (8) flexibles et la saillie d'accouplement à baïonnette (10) qui, dans la position de verrouillage, est logée dans une fenêtre pour accouplement à baïonnette (17) de l'embout femelle (3).

4. Raccord rapide selon la revendication 1, 2 ou 3, **caractérisé en ce que** le téton de blocage (8) est prévu au niveau d'un étrier (5) flexible.

5. Raccord rapide selon la revendication 3 ou 4, **caractérisé en ce que** l'insert (4) comporte non seulement deux saillies d'accouplement à baïonnette (10) agencées symétriquement l'une en face de l'autre, mais aussi deux tétons de blocage (8), les tétons de blocage et les saillies d'accouplement à baïonnette étant décalés de 90° les uns par rapport aux autres et les tétons de blocage étant disposés sur un plus grand diamètre que les saillies d'accouplement à baïonnette.

6. Raccord rapide selon la revendication 5, **caractérisé en ce que** le boîtier extérieur (2) de l'embout mâle (1) et l'embout femelle (3) sont plats, de telle sorte que, dans la position de verrouillage, ils présentent une forme plane, lisse se prolongeant l'un dans l'autre et **en ce que** les tétons de blocage (8) et les cavités (12) correspondantes sont disposés sur le plus grand diamètre de la forme plane.

7. Raccord rapide selon la revendication 5 ou 6, **caractérisé en ce que** les tétons de blocage (8), symétriquement l'un en face de l'autre, sont prévus sur les extrémités des branches latérales (6) d'un étrier (5, 6) en forme de U de l'insert (4), et **en ce que** la base (5) de l'étrier en U comporte des encoches (7) pour la suspension élastique des tétons de blocage.

8. Raccord rapide selon l'une quelconque des revendications précédentes, **caractérisé en ce que** les tétons de blocage (8) sont conçus en forme de toit, dont le faîte est orienté vers la cavité de l'embout femelle (3).
